# EUROPEAN PATENT APPLICATION

(11) **EP 2 478 861 A2**
(43) Date of publication of application: **25.07.2012**
(21) Application number: 12152263.5
(22) Date of filing: 24.01.2012
(51) Int. Cl.: A61B 18/12

(54) **Reusable medical device with advanced counting capability**

(30) Priority: 24.01.2011 US 201113012629
(71) Applicant: Tyco Healthcare Group, LP, Mansfield, MA 02048 (US)
(72) Inventor: Allen, Charles D., Broomfield, Colorado 80020 (US)
(74) Representative: HOFFMANN EITLE

(57) **Abstract**

An electrosurgical instrument including a housing assembly having a first disposable treatment portion selectively attached thereto. The disposable treatment portion is adapted to connect to an electrosurgical generator that supplies energy to the electrosurgical instrument. A control circuit is included and disposed in electrical communication between the electrosurgical generator and the first disposable treatment portion. The control circuit is selectively actuatable to enable the flow of electrosurgical energy from the electrosurgical generator to the first disposable treatment portion. An indicator, coupled to the housing assembly or the first disposable treatment portion, is configured to indicate a parameter related to the use of the electrosurgical instrument. The control circuit is configured to prevent activation of the electrosurgical instrument when the parameter of the indicator indicates the useful life of the disposable treatment portion has expired.

## Description

### BACKGROUND

### 1. Technical Field

The present disclosure relates to a limited use reusable electrosurgical instrument and more particularly, the present disclosure relates to systems and methods for tracking and controlling the use of electrosurgical instruments and reusable portions thereof.

### Background of Related Art

The use of electrosurgical instruments is well known in the art. Electrosurgical instruments typically utilize both mechanical clamping action and electrical energy to affect hemostasis by heating the tissue and blood vessels to coagulate, cauterize and/or seal tissue. Over the course of a surgical procedures a clinician may use a variety of electrosurgical instruments such as, for example, elongated electrosurgical forceps to cauterize, coagulate/desiccate and/or to simply reduce or slow bleeding in a surgical cavity, electrosurgical pencil or scalpel for cutting or to cauterize a surgical opening, an electrosurgical vessel sealing device including actuating jaw members of an end effector assembly for sealing and cutting vessels. In addition to the assortment of electrosurgical instruments available to a clinician, many electrosurgical instrument are configured to receive a variety of attachments or members, such as tips, jaws, blades, electrode configurations or combination thereof.

During the course of a surgical procedure, a clinician may employ a variety of different instrumentation, including reusable instruments, limited use reusable instruments, and disposables instrument. Reusable instruments are instrument where the reusability of the instrument is limited only by operability of the instrument (i.e., proper operation, recommended maintenance and/or reconditioning schedules). A limited use reusable instrument includes instruments with a limited useful life, wherein the useful life of the instrument may be based the number of electrical activations, the cumulative time of electrical activation, the number of mechanical activations, the number of surgical procedures performed or any combination thereof. A disposable instrument is an instrument intended to be introduced to a surgical field, used for its intended purpose and immediately disposed of thereafter.

Instruments may also be configured to receive a disposable or limited use attachment or member. For example, a reusable electrosurgical scalpel may be configured to receive a limited use or disposable blade or a limited use reusable vessel sealing device may be configured to receive a disposable single-use shaft and end effector or end effector assembly or jaw assembly.

A manufactures "limited use" or "disposable" recommendation is typically based on performance testing, reliability testing, the inability to properly sterilize the instrument or attachment member using conventional sterilizing techniques, the material degradation as a result of conventional sterilization techniques or any combination thereof A clinician must know the use limitation, track the actual usage and abide by the instructions by disposing of the instrument after the actual usage has been exceeded. In some instances there may be a temptation to re-use disposable instruments or use items beyond the recommended useful life to save costs especially in clinic-type environments or low-income areas. Obviously, health issues and concerns arise when disposable instruments are re-used for surgical purposes or when instruments are used beyond their recommended life or cycles. As such, the use recommendation for reusable or disposable members sometimes depends on the clinician, surgeon or surgical personnel to discard the instrument or member after the manufactures recommended number of uses is exceeded.

To assist clinicians in abiding by a manufacturer's intended use and disposal instructions, and to prevent intentional re-use of instruments, manufactures have employed a number of systems and methods. For example, to prevent accidental reuse, some instruments are packaged such that the packaging is destroyed when opened. Other instruments employ smart-connectors to prevent reconnection and reuse of a particular instrument with the same electrosurgical generator. Other instruments have employed a time-out device configured to prevent re-use of the electrosurgical instrument after a predetermined time limit.

### SUMMARY

The present disclosure describes an electrosurgical instrument configured to receive electrosurgical energy from an electrosurgical generator. The electrosurgical instrument includes a housing assembly having a first disposable treatment portion selectively attached thereto, a control circuit and at least one indicator coupled to the housing and/or the disposable treatment portion. The first disposable treatment portion connects to an electrosurgical generator that supplies energy thereto. The control circuit is disposed between the electrosurgical generator and the first disposable treatment portion and is selectively actuatable to enable the flow of electrosurgical energy from the electrosurgical generator to the first disposable treatment portion. The indicator indicates a parameter related to the use of the electrosurgical instrument and the control circuit prevents activation of the electrosurgical instrument when the parameter indicates the useful life of the first disposable treatment portion has expired. The parameter may be related to actuations of the first disposable treatment portion, related to the useful life of the housing assembly and/or related to a near end-of-life condition. The first disposable treatment portion may include an actuating jaw mechanism or an end effector assembly with an actuating jaw mechanism.

The control circuit may enable use of the electrosurgical instrument upon replacement of the first disposable treatment portion with an unused subsequent disposable treatment portion different than the first disposable treatment portion. The control circuit may also include circuitry to temporarily or permanently disable the flow of electrosurgical energy to the first disposable treatment portion and/or a subsequent disposable treatment portion. The control circuit may include a sensor(s) that detects mechanical actuation of the trigger assembly, mechanical actuation of a jaw closure assembly, the removal of the first disposable treatment portion and/or the insertion of an unused subsequent disposable treatment portion different than the first disposable treatment portion. The control circuit may also include circuitry to disable the actuation signal from the electrosurgical instrument and provided to the electrosurgical generator.

An indicator may indicate a first parameter related to the useful life of the first disposable treatment portion and/or a subsequent disposable treatment portions. Another indicator may indicate a second parameter related to the useful life of the housing assembly, actuations of the electrosurgical instrument, replacements of the first disposable treatment portion and/or the insertion of subsequent disposable treatment portions. The second parameter may also indicate the end-of-life of the housing assembly after a predetermined number of replacements exceeds a predetermined limit.

The control circuit may be configured to indicate a parameter related to an end-of-life condition and/or a near end-of-life condition. The parameter may indicate a numerical indicator and/or a color indicator. The numerical indicator may be related to an end-of-life condition and the color indicator may be related to near end-of-life condition. The control circuit may be configured to indicate a parameter related to a near end-of-life condition prior to indicating an end of useful life parameter.

The present disclosure also describes an electrosurgical instrument that includes a housing assembly having a first disposable treatment portion selectively attached thereto, a control circuit disposed between the electrosurgical generator and the disposable treatment portion and an indicator coupled to the housing assembly and configured to indicate a parameter related to the use of the electrosurgical instrument. The first disposable treatment portion connects to an electrosurgical generator that supplies energy thereto. The control circuit is selectively actuatable to enable the flow of electrosurgical energy from the electrosurgical generator to the disposable treatment portion. The control circuit may also be configured to prevent activation of the electrosurgical instrument when the parameter of the indicator indicates the useful life of the housing assembly has expired.

The present disclosure also describes an electrosurgical instrument that includes a housing assembly adapted to connect to an electrosurgical generator that supplies electrosurgical energy thereto and a first disposable treatment portion selectively attached to a distal end of the housing assembly and configured to receive electrosurgical energy therefrom. The first disposable treatment portion includes an actuating jaw mechanism that delivers electrosurgical energy to tissue, a control circuit operably associated with the housing assembly and the actuating jaw mechanism and an indicator operably coupled to the first disposable treatment portion that indicates a parameter related to the use of the disposable treatment portion. The control circuit is selectively actuatable to enable the flow of electrosurgical energy from the electrosurgical generator to the actuating jaw mechanism and configured to prevent activation of the electrosurgical instrument when the parameter indicates the useful life of the first disposable treatment portion has expired.

The electrosurgical instrument may further include a third indicator, coupled to the housing assembly or coupled to the first disposable treatment portion, that indicates a near end-of-life condition. The third indicator may indicate a near end-of-life condition prior to the first indicator or the second indicator indicating the expiration of useful life.

The present disclosure also describes a limited use disposable end effector assembly including a shaft with a proximal end and a distal end, an actuating jaw mechanism connected to the distal end of the shaft that delivers electrosurgical energy received from the shaft to tissue, a control circuit disposed in the shaft between the electrosurgical instrument housing assembly and the actuating jaw mechanism and an indicator. The proximal end of the shaft is configured to selectively attached to a distal end of an electrosurgical instrument housing assembly and is configured to selectively receive electrosurgical energy from the electrosurgical instrument housing assembly. The control circuit is selectively actuatable to enable the flow of electrosurgical energy to and from the actuating jaw mechanism. The indicator is coupled to the shaft and configured to indicate a parameter related to the use of the limited use disposable end effector assembly. The control circuit prevents activation of the actuating jaw mechanism when the parameter indicates the useful life of the limited use disposable end effector assembly has expired.

The present disclosure also describes an electrosurgical system that includes a source of electrosurgical energy and an electrosurgical instrument. The electrosurgical instrument includes a housing assembly that connects to a source of electrosurgical energy, a first disposable treatment portion that releasably attaches to the distal end of the housing assembly and is in electrical communication with the electrosurgical generator through the housing assembly and a control circuit operably associated with the electrosurgical generator and the first disposable treatment portion. A first indicator is coupled to the housing assembly that indicates a first parameter related to the use of the housing assembly and a second indicator is coupled to the first disposable treatment portion that indicates a second parameter related to the use of the first disposable treatment portion. The control circuit is selectively actuatable to enable the flow of electrosurgical energy from the electrosurgical generator to the first disposable treatment portion. The control circuit may also prevent activation of the electrosurgical instrument when the first and/or second parameters indicates the useful life of one of the housing assembly and/or the first disposable treatment portion has expired.

The first disposable treatment portion may include an actuation jaw mechanism or an end effector assembly having an actuating jaw mechanism.

The first parameter may be related to actuations of the housing assembly and the second parameter may be related to the useful life of the first disposable treatment portion. The control circuit may disable the electrosurgical system when the second parameter indicates the useful life of the first disposable treatment portion has expired. The control circuit may enable the electrosurgical instrument upon replacement of the first disposable treatment portion with an unused subsequent disposable treatment portion different than the first disposable treatment portion.

In another embodiment, the second parameter may be related to the useful life of the first disposable treatment portion and the first parameter indicates the number of subsequent unused disposable treatment portion replacements the housing assembly may receive. The control circuit may permanently disable the flow of electrosurgical energy through the electrosurgical instrument when the first parameter meets a predetermined condition.

In yet another embodiment, the control circuit may include a sensor that detects mechanical actuations of the trigger assembly, a sensor that detects mechanical actuations of the jaw closure assembly and/or a sensor that detects the removal of the first disposable treatment portion and/or the insertion of an unused subsequent disposable treatment portion different than the first disposable treatment portion.

The present disclosure also describes methods of performing an electrosurgical procedure. One method includes the steps of: providing an electrosurgical device in an inoperable condition; making the device operable by replacing a spent end effector assembly with an unused end effector assembly different than the spent end effector assembly; transferring information from the unused end effector assembly to a control circuit in the electrosurgical device; enabling the electrosurgical device if the information from the unused end effector assembly indicates the unused end effector assembly is capable of performing electrosurgical procedures, and performing at least one electrosurgical procedure with the unused end effector assembly. The method may also include the step of indicating an indicator related to the information provided from the unused end effector.

Another method of performing an electrosurgical procedure includes the steps of: providing an electrosurgical instrument with a housing assembly selectively attached to an end effector assembly, the electrosurgical instrument being in an inoperable condition; determining if the inoperable condition is the result of a spent end effector; removing the spent end effector from the housing assembly; sensing the removal of the spend end effector from the housing assembly and reporting the removal to a control circuit; inserting an unused end effector assembly different that the spent end effector assembly; sensing the insertion of the unused end effector assembly and reporting the insertion to the control circuit, and enabling the electrosurgical instrument. The method may also include the steps of: providing an indicator to indicate a parameter related to the replacement of the spent end effector assembly; determining if the electrosurgical instrument is capable of receiving an unused end effector assembly, and permanently disabling the electrosurgical instrument if the electrosurgical instrument is not capable of receiving an unused end effector assembly.

### BRIEF DESCRIPTION OF THE DRAWINGS

Various embodiments of the subject instrument are described herein with reference to the drawings wherein:

Fig. 1 is a perspective view of a bipolar forceps, shown in an open configuration, including a housing assembly, a end effector assembly and a use indicator system according to the present disclosure;

Fig. 2 is a perspective view of the bipolar forceps of Fig. 1 shown in closed configuration;

Fig. 3 is a side perspective view of the bipolar forceps of Fig. 1 shown with the trigger and handle actuated, with partial cross-sections exposing internal components of the forceps and components of the use indicator system;

Fig. 4A is a perspective view of the bipolar forceps of Fig. 1 with a use indicator indicating a spent end effector assembly;

Fig. 4B is a perspective view of the bipolar forceps of Fig. 4A illustrating the removal of the spent end effector assembly and subsequent insertion of an unused end effector assembly;

Fig. 4C is a perspective view of the bipolar forceps of Fig. 4B assembly with the unused end effector assembly;

Fig. 5A is a perspective view of a bipolar forceps having a spent end effector assembly;

Fig. 5B is a perspective view of the bipolar forceps of Fig. 5A illustrating the removal of the spent end effector assembly and subsequent insertion of an unused end effector assembly; and

Fig. 5C is a perspective view of a bipolar forceps of Fig. 5B assembly with the unused end effector assembly.

### DETAILED DESCRIPTION

In the drawings and in the descriptions which follow, the term "proximal," as is traditional, will refer to the portion of the instrument closer to the user, while the term "distal" will refer to the end of the instrument farther from the user.

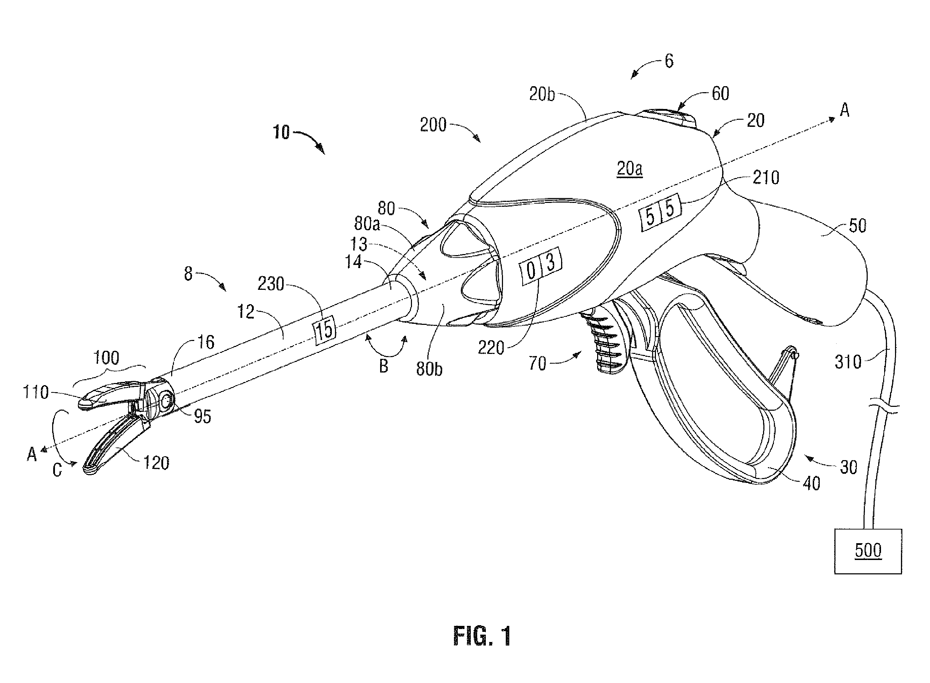

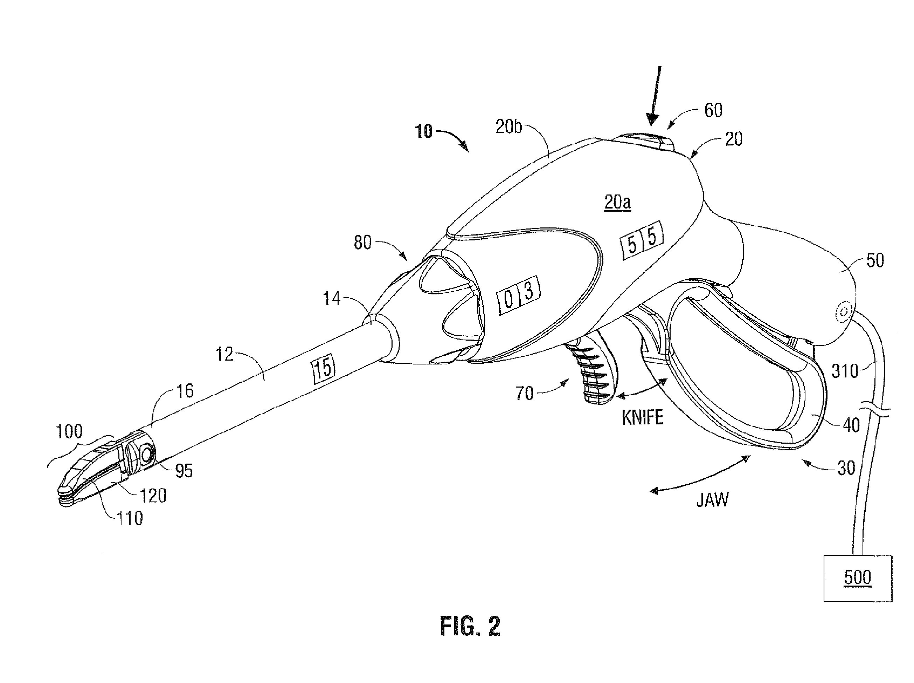

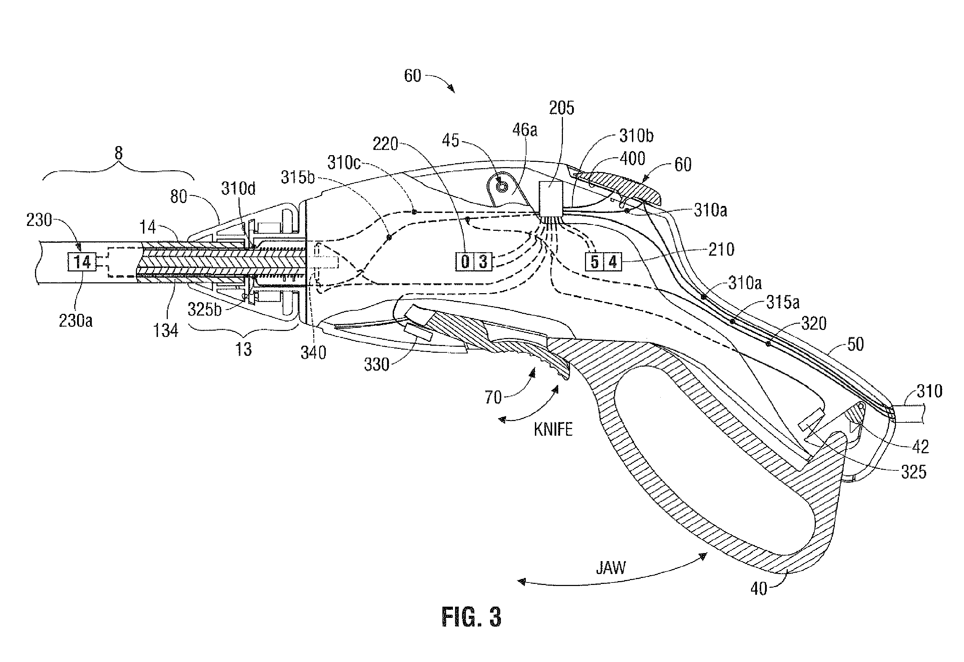

Turning now to Figs. 1 and 2, one embodiment of a bipolar forceps 10 (i.e., forceps 10) is shown for use with various surgical procedures and generally includes a housing assembly 6, a shaft and end effector assembly 8 and a use indicator system 200. The housing assembly 6 and the shaft and end effector assembly 8 mutually cooperate to grasp, seal and divide large tubular vessels and large vascular tissues. The use indicator system 200 is integrated into the housing assembly 6 and/or the shaft and end effector assembly 8 and includes control circuitry 205 (see Fig. 3) and one or more use indicators 210, 220, 230. Control circuitry 205 is configured enable and/or disable the delivery of electrosurgical energy based on the use of the housing assembly 6 or any portion thereof, the use of the shaft and end effector assembly 8 or any portion thereof. The indicators 210, 220, 230 are controlled by the control circuitry and are each configured to indicate at least one parameter related to the use of the of the forceps 10.

Although the majority of the figure drawings depict a forceps 10 with an indicator system 200 for use in connection with vessel sealing and endoscopic surgical procedures, the present disclosure, systems and methods described herein may be used for any electrosurgical instruments, such as, for example, an electrosurgical pencil, an ablation device, an electrosurgical scalpel, an electrosurgical coagulation device and an electrosurgical cauterization device. These other types of electrosurgical surgical instruments may be configured to incorporate one or more aspects of the present disclosure. For the purposes herein, the forceps 10 with a use indicator system 200 is described in terms of an open instrument; however, it is contemplated that an endoscopic version of the forceps may also include the same or similar operating components and features as described below.

In addition, while the shaft and end effector assembly 8 depicted in the figure drawings includes a shaft 12 and an end effector 100 for vessel sealing, the present disclosure, systems and methods described herein may be used for any disposable or partially disposable end effector, such as, for example, blades, electrodes, antennas, tips or any combination thereof.

The housing assembly 6 of the forceps 10 includes a housing 20, a handle assembly 30, a rotating assembly 80 and a trigger assembly 70. The housing 20 includes first and second housing halves, 20a and 20b, respectively, that form or house various drive systems and components of the handle assembly 30, rotating assembly 80, trigger assembly 70 and use indicator system 200. Handle assembly 30 includes a fixed handle 50 and a movable handle 40. Fixed handle 50 is integrally associated with housing 20 and movable handle 40 is movable relative to fixed handle 50 as explained in more detail below with respect to the operation of the forceps 10. Rotating assembly 80 is operatively associated with the housing 20 and is rotatable about a longitudinal axis "A-A" defined through the forceps 10. Details of the rotating assembly 80 are described in more detail with respect to U.S. Patent No. 7,766,910 entitled "VESSEL SEALER AND DIVIDER FOR LARGE TISSUE STRUCTURES".

End effector assembly 100 includes opposing first and second jaw members 110, 120, respectively, that cooperate to effectively grasp tissue for sealing purposes, and a knife assembly (not explicitly shown) including an actuating knife (not explicitly shown) for severing tissue sealed between the first and second jaw members 110, 120. End effector assembly 100 is designed as a bilateral assembly (i.e., first and second jaw members 110, 120 pivot relative to one another about a pivot pin 95 disposed therethrough). The first and second jaw members 110, 120, respectively, may be curved to facilitate manipulation of tissue and to provide better "line of sight" for accessing organs and large tissue structures.

Trigger assembly 70 mounts atop movable handle 40 and cooperates with the knife assembly (not explicitly shown) to selectively translate knife (not explicitly shown) through a tissue seal formed between first and second jaw members 110, 120, respectively. The trigger assembly 70 is initially prevented from firing by the locking flange (not explicitly shown) disposed on movable handle 40 which abuts against the trigger assembly 70 prior to actuation of the movable handle 40. Details relating to the operation of the trigger assembly 70 are described in more detail in U.S. Patent No. 7,766,910 to Hixson et al..

Rotating assembly 80 includes two C-shaped first and second halves 80a, 80b, respectively, which, when assembled, form the rotating assembly 80. The first and second halves 80a, 80b, respectively, cooperate to matingly engage shaft 12 to permit selective rotation of the shaft 12 about axis "A-A" by manipulating the rotating member 80 in the direction of the arrow "B", which, in turn, rotates the end effector assembly 100 in the direction of arrow "C".

Shaft 12 has a distal end 16 dimensioned to engage the end effector assembly 100 and a proximal end 14 dimensioned to engage the housing assembly 6. Proximal end 14 of shaft 12 may further include an end effector connector 13 that electrically connects various electrical connections between the effector assembly 100 with the housing assembly 6 and mechanically engages the shaft 12 with various mechanical systems between end effector assembly 100 and the housing assembly 6. Details of how the shaft 12 electrically connects and mechanically couples to the end effector assembly 100 and housing assembly 6 are described in more detail with respect to U.S. Patent No. 7,766,910 to Hixson et al.

With respect to actuation of the instrument, movable handle 40 is selectively movable about a pivot pin 45 (see Fig. 3) from a first position spaced away from the fixed handle 50, as illustrated in Fig. 1, to a second position in closer proximity to the fixed handle 50, as illustrated in Fig. 2. Movable handle 40 of handle assembly 30 is ultimately connected to a drive assembly (not explicitly shown) that imparts movement of the first and second jaw members 110, 120, respectively, from an open position wherein the first and second jaw members 110, 120 are disposed in spaced relation relative to one another, to a clamping or closed position wherein the first and second jaw members 110, 120, respectively, cooperate to grasp tissue therebetween. A more detailed explanation of the inter-cooperating components of the handle assembly 30 and the drive assembly (not explicitly shown) and the actuation of the shaft 10 and end effector assembly 100 is described in more detail with respect to U.S. Patent No. 7,766,910 to Hixson et al.

Forceps 10 also includes an electrosurgical cable 310 that connects the forceps 10 to a source of electrosurgical energy, e.g., a generator 500 (shown schematically). It is contemplated that generators such as those sold by Covidien - Engery Based Devices, located in Boulder, Colorado may be used as a source of electrosurgical energy, e.g., Ligasure^{™} Generator, ForceTriad^{™} Electrosurgical Generator, Force EZ^{™} Electrosurgical Generator, Force FX^{™} Electrosurgical Generator, Force^{™} 1C, Force^{™} 2 Generator, SurgiStat^{™} II or other envisioned generators which may perform different or enhanced functions. The generator 500 includes various safety and performance features including isolated output, independent activation of accessories.

Turning now to Fig. 3, cable 310 is internally divided into cable leads 310a, 315a and 320 which are designed to transmit electrical potentials through their respective feed paths through the forceps 10. Details relating to the electrical connections are explained in more detail below with respect to the above-identified patent application or, alternatively, with respect to U.S. Patent No. 7,789,878 entitled "IN-LINE VESSEL SEALER AND DIVIDER".

Once the desired position for the sealing site is determined and the first and second jaw members 110, 120, respectively, are properly positioned, movable handle 40 may be compressed fully such that movable handle 40 engages a catch 42 (See Fig. 3) and locks relative to fixed handle 50, which, in turn, locks the first and second jaw members 110, 120, respectively, in a closed position against the tissue (see Fig, 2). At this point the first and second jaw members 110, 120, respectively, are fully compressed about the tissue (not explicitly shown) positioned between the first and second jaw members 110, 120. Moreover, the forceps 10 are now ready for selective application of electrosurgical energy by actuating a switch 60, and subsequent separation of the tissue along the formed tissue seal, by actuating the trigger 70.

More particularly, by controlling the intensity, frequency and duration of the electrosurgical energy applied to the tissue, the user can treat tissue (i.e., seal tissue). As energy is being selectively transferred between the first and second jaw members 110, 120 of the end effector assembly 100 and through the tissue, a tissue seal forms isolating two tissue halves. When activated via the trigger assembly 70, the knife (not explicitly shown) progressively and selectively divides the tissue along an ideal tissue plane in a precise manner to effectively and reliably divide the tissue into two sealed halves.

Switch 60 is ergonomically dimensioned and conforms to the outer shape of housing 20 (once assembled). Switch 60 is designed to electromechanically cooperate with internal circuitry, discussed hereinbelow, to allow a user to selectively activate the first and second jaw members 110, 120, respectively. Switch 60 permits the user to selectively activate the forceps 10 in a variety of different orientations, i.e., multi-oriented activation or toggle-like activation.

With reference to Fig. 3, switch 60, when depressed, connects first trigger lead 310a to the second trigger lead 310b through the switch. Second trigger lead 310b connects to the third trigger lead 310c through the control circuit 205 and third trigger lead 310c carries the first electrical potential to the first jaw member 110 through the shaft 12 (or portion therethrough, thus completing one leg of the bipolar circuit. In the second leg of the bipolar circuit, the first return lead 315a connects to the second return lead 315b through the control circuit 205 and the second return lead 315b connects to the second jaw member 120. In another embodiment, switch 60 and control circuit 205 are protected e.g., removed from the actual current loop, that supplies electrical energy to the first and second jaw members 110, 120, respectively, thereby reducing the chances of electrical failure of the switch 60 and/or control circuit 205 due to high current loads during activation.

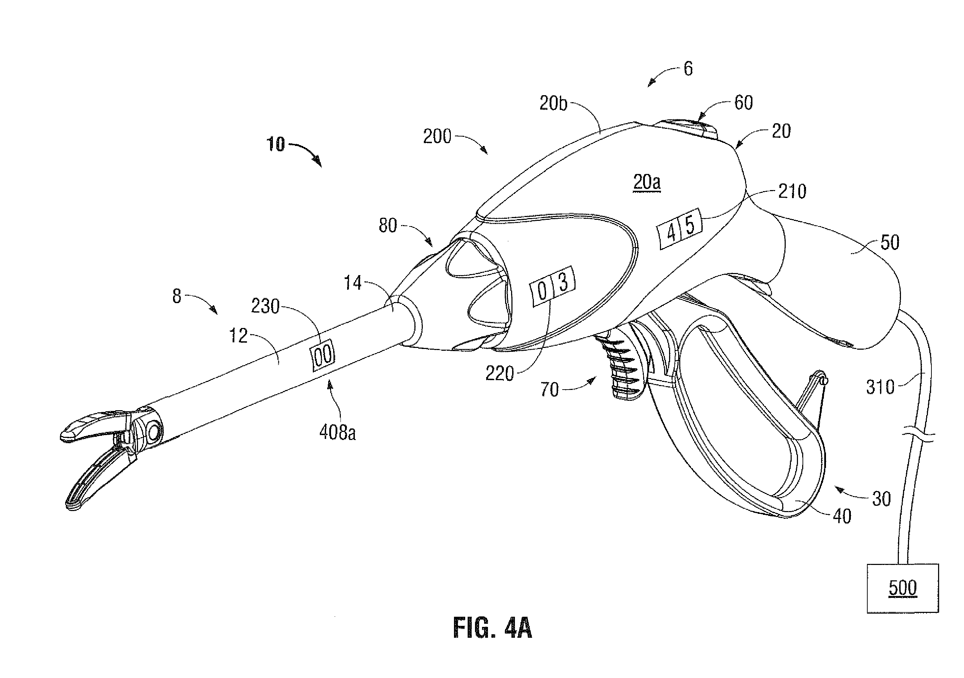

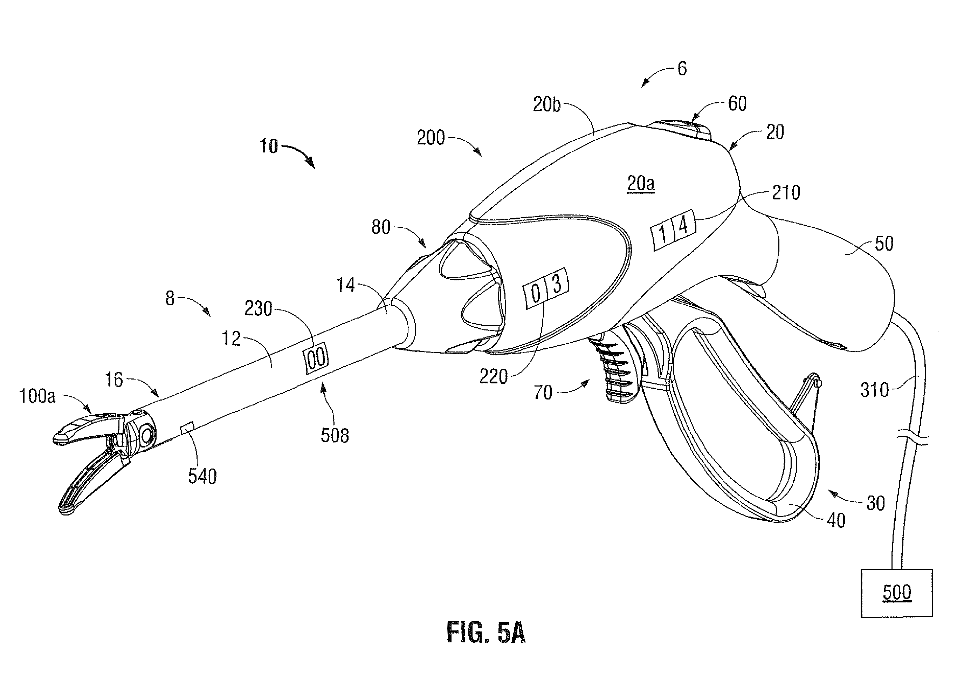

The forceps 10 are designed such that various components are fully or partially disposable depending upon a particular purpose or to achieve a particular result. For example, housing assembly 6 may be disposed of after a surgical procedure and/or after a predetermined number of actuations while the shaft and end effector assembly 8 (or either portion thereof) may be replaced one or more times during the same procedure (e.g., after a predetermined number of actuations; See Figs. 4A - 4B). In another embodiment, the end effector assembly 100 may be selectively and releasably engageable with the distal end 16 of the shaft 12 and may be discarded after a the predetermined number of actuations (See Figs. 5A - 5B). End effector assembly 100, and in particular the knife (not explicitly shown) that requires a sharpened surface to effectively cut tissue, may have a limited number of actuations as compared to the housing assembly 6.

In another embodiment, the proximal end 14 of shaft 12 or the end effector connector 13 connected to the proximal end 14 of shaft 12 may be selectively and releasably engageable with the handle assembly 30 or a portion thereof. In either of these two instances, the forceps 10 would be considered "partially disposable" or "reposabie", e.g., a new or different shaft and end effector assembly 8 (or end effector assembly 100) selectively replaces the spent shaft and end effector assembly 8 (or end effector assembly 100) as needed.

The use indicator system 200 includes one or more indicators and a control circuit 205 configured to track one or more specific occurrences related to the operation of the forceps 10. The forceps 10 illustrated in Figs. 1-3 and 5 includes a control circuit 205, a handle activation indicator 210, an end effector replacement indicator 220 and an end effector activation indicator 230. Any combination of these elements is contemplated. The control circuit 205 is configured to recognizes and track one or more events related to the forceps 10 or configured to recognize a specific condition related to the forceps 10. For example, events or conditions recognized and/or tracked by the control circuit 205 may include the clamping/closing of the first and second jaw members 110, 120, the electrical activation, a measured temperature of one or more components exceeding a threshold value, actuating and/or transition of the knife (total actuations or actuations with positive effect on tissue, e.g., transitions after electrical activation of forceps 10), the initiation of a sealing cycle, the completion of a sealing cycle or any combination or accumulation of events and/or conditions.

Control circuit 205 connects between second and third trigger leads 310b, 310c, between first and second return leads 315a, 315b and any electrical signal related to providing electrosurgical power. Control circuit 205 may monitor the signals related to delivery of the electrosurgical energy and control the flow of electrosurgical energy directly or indirectly. For example, direct monitoring of the signals may include connecting the current carrying conductors through the control circuit 205 wherein the control circuit 205 directly measures energy delivery through the forceps 10. Direct control may include interrupts to the flow of energy by opening the electrosurgical energy delivery circuit. Indirect monitoring may include monitoring the switch 60, or the physical position of the switch, to determine when the switch 60 is depressed. Indirect control may include opening the signal from the switch 60 thereby preventing the generator 500 from receiving an enable signal from the switch 60 or providing a fault control signal to the generator 500 thereby preventing the generator from delivering electrosurgical energy.

Housing assembly 6 may also house one or more electrical, mechanical or electro-mechanical sensors configured to provide the control circuit 205 with feedback related to the position of the handle assembly 30, the position of the knife assembly 70 and/or the presence (or lack thereof) of a shaft and end effector assembly 8 or an end effector assembly 100 or any portion thereof. Closure of the movable handle 40 with respect to the fixed handle 50 drives the catch 42 into a jaw position sensor 325 thereby providing the control circuit 205 with an indication that the end effector assembly 100 is closed. Closure of the trigger assembly 70 contacts a knife extend sensor 330 thereby providing the control circuit 205 with an indication that the knife (not explicitly shown) in the end effector assembly 100 is extended. Replacement of a shaft and end effector assembly 8 is indicated by a shaft end effector assembly sensor 340 (e.g., cycling of the signal from the shaft and end effector assembly sensor 340 indicates the removal and insertion of a new shaft and end effector assembly 8) and provided to the control circuit 205. Alternatively, the shaft and end effector assembly 8 may include a end effector assembly position sensor 540 in the distal end 16 of the shaft 12 that connects to the control circuit 205 via a suitable electrical connection (see end effector assembly position sensor 540, Fig. 5A-5C).

In one embodiment, the control circuit 205 tracks the number of handle assembly 30 activations and displays a parameter related to the activations of the handle assembly 30 on a handle activation indicator 210. The parameter related to activations of the handle assembly 30 may include the clamping of the first and second jaw members 110, 120, the electrical activation of the sealing section, a measured temperature exceeding a threshold value, activating and/or extending the cutting knife (not explicitly shown) in the end effector assembly 100, initiation of a sealing cycle, completion of a sealing cycle or any combination or accumulation of events or conditions. The parameter displayed on the handle activation indicator 210 may be a graphical display or numerical count-down of the remaining handle assembly 30 activations.

In another embodiment, the control circuit 205 tracks the number of new shaft and end effector assembly 8 replacements. The useful life of the housing assembly 6 may be limited by the number of activations as discussed hereinabove or may be limited by a predetermined number of shalt and end effector assembly 8 replacement cycles (i.e., the number of times an unused shaft and end effector assembly 8, or portion thereof, is connected to the housing assembly 6). Limiting the use of the housing assembly 6 to a predetermined number of replacement cycles limits the possibility of a disposable housing assembly 6 being used beyond a safe range of operation and/or may limit the possibility that a disposable housing assembly 6 is utilized for a second surgery. The parameter displayed on the end effector replacement indicator 220 may be a graphical display or numerical count of shaft and end effector assembly 8 replacements or may be a graphical display or numerical count-down of the remaining shaft and end effector assembly 8 replacements.

The useful life of a particular shaft and end effector assembly 8, or portion thereof, may be limited to a specific number of activations and the control circuit 205 many track and limit the use (i.e., the number of shaft and end effector assembly 8 activations) of the current shaft and end effector assembly 8. The shaft and end effector assembly 8 activations may include the clamping of the first and second jaw members 110. 120, the electrical activation of the sealing section of the forceps 10, a temperature on the forceps exceeding a threshold value, activation and/or the transition of the cutting knife (not explicitly shown) in the end effector assembly 100 of the forceps 10, the initiation of a sealing cycle, the completion of a sealing cycle or any combination or accumulation of events and/or conditions.

The forceps 10 may be temporarily disabled or permanently disabled based on past usage. For example, a particular housing assembly 6 may be capable of performing 45 actuations and receiving up to three (3) shaft and end effector assembly 8 replacements before the performance is no longer acceptable; each shaft and end effector assembly 8 may be capable of performing fifteen (15) actuations. The control circuit 205 monitors the number of actuations for the housing assembly 6 and the number of actuations of the shaft and end effector assembly 8. As discussed hereinabove, the number and character of an "actuations" for the housing assembly 6 and the shaft and end effector assembly 8 need not be the same. When the control circuit 205 determines that the shaft and end effector assembly 8 is spent (i.e., performed the maximum number of actuation), the control circuit 205 temporarily disables the housing assembly 6 until the spent shaft and end effector assembly 8 (or portion thereof, i.e., end effector assembly 100) has been replaced with an unused shaft and end effector assembly 8. Replacing a shaft and end effector assembly 8 decreases by one the number of shaft and end effector assembly 8 replacements.

Each of the indicators (e.g., handle activation indicator 210, end effector replacement indicator 220 and/or end effector activation indicator 230) may provide an end-of-life indicator, wherein the control circuit 205 temporarily or permanently disables the operation of the forceps 10 when the end-of-life is indicated. For example, the control circuit 205 may temporarily disable the forceps 10 if the shaft and end effector assembly 8 has exceeded the maximum number of activations. The end-of-life indicator 210, 220 for the housing assembly 6 may be re-enabled after the spent shaft and end effector assembly 8 is replaced with an unused shaft and end effector assembly 8.

In another embodiment, the control circuit 205 determines a condition or series of activations or actuations that require the housing assembly 6 and/or shaft and end effector assembly 8 to be prematurely disabled (i.e., disabled before the predetermined number of actuations are exceeded). For example, control circuit 205 may track the number of electrosurgical sealing activations that occur without an actuation of the knife (not explicitly shown) thereby indicating that the forceps 10 is not providing an adequate seal for cutting or indicating that the forceps is not being utilized for its intended use.

In another embodiment, an unused shaft and end effector assembly 8, when inserted into the housing assembly 6, transfers one or more parameters related to the unused shaft and end effector assembly 8 to the control circuit 205. One parameter may include one or more unique identifiers used to identify that specific shaft and end effector assembly 8, such as, for example, past usage, a manufacturing timestamp information, calibration parameters, test data, expiration data and/or one or more parameters related to intended use. Another parameter may include one or more operational parameters such as, for example, the number of available activations, energy delivery specifications and limitations and compatibility data. The control circuit 205 may utilize the one or more of the parameters received from the shaft and end effector assembly 8 to updating one or more indicators 210, 220, 230. Control circuit 205 may provide one or more the parameters to the generator 500 or may use one or more of the parameters in an algorithm, the results, of which, are provided to the generator 500.

Control circuit 205 may also send one or more parameters to the shaft and end effector assembly 8. One parameter may include information related to the present usage, such as, the number of activations or a parameter that indicates how long the shaft and end effector assembly 8 has been connected to the housing assembly 6.

Usage may be indicated as an accumulation of the number of activations or may be indicated as the number of activations remaining. The indicator may have an initial value of zero and the indicator may be incremented for each identified occurrence and/or activation. The device (or any portion thereof) may be disabled (temporarily or permanently) when the value of the indicator meets or exceeds a threshold value. Alternately, the indicator may be preset to a value indicating the number of occurrences and/or activations available for the particular device. The indicator value is decremented for each identified occurrence and/or activation and the device is disabled (temporarily or permanently) when the indicator value meets a condition related to a threshold value. The threshold value may be predetermined prior to use or may be determined and/or calculated when the forceps is placed into service. For example, the control circuit 205 may read the threshold value from the forceps or shaft and end effector assembly 8, the control circuit 205 may determine a threshold value based on the identification of the shaft and end effector assembly 8 or the control circuit 205 may determine a value based on the identification of the shaft and end effector assembly 8 mated with the particular housing assembly 6 (i.e., the threshold value for a particular shaft and end effector assembly 8 may be determined by the housing assembly 6 to which it the shaft and end effector 6 is installed).

In yet another embodiment of the present disclosure, an indicator system 200 as described herein includes one or more mechanical indicators to indicate the number of actuations of the housing assembly 6 (or portion thereof) and/or actuations of the shaft and end effector assembly 8 (or any portion thereof). For example, a mechanical indicator may be integrated into the drive system for the trigger assembly 70, handle assembly 30 and/or the switch 60 such that an activation thereof decrements the mechanical indicator. The mechanical indicator may permanently disable or temporarily disable the housing assembly 6, or any part thereof, or may permanently disable or temporarily disable the shaft and end effector assembly 8, or any portion thereof, when the mechanical indicator indicates that the end-of-life has been reached.

The mechanical indicator may be any suitable mechanical device configured to count up (or count down) the number of mechanical actuations, such as, for example, a mechanical tally counter, a mechanical ratchet driven counter, a mechanical stroke counter or a mechanical revolution counter. Mechanical indicator may be configured to temporarily or permanently lock the drive, or any component thereof, thus preventing any further actuations of the device (or any portion thereof) when the number of activations exceeds a predetermined number of actuations as discussed herein with respect to an electrical counter arrangement.

Mechanical indicators may be configured to be reset (e.g., allowing additional or subsequent actuations). The reset of the mechanical indicator may be effectuated by an intervening event, occurrence or action. For example, the removal or insertion of a new shaft and end effector assembly 8, as described herein, may mechanically reset one or more mechanical indicators thereby allowing further operation of the forceps 10.

In yet another embodiment, the number of mechanical reset occurrences may be limited by a mechanical reset limiter. For example, after the maximum number of replacement shaft and end effector assemblies 8 are spent the mechanical reset indicator may prevent the removal of the last spend shaft or may prevent the subsequent insertion of an unused shaft and end effector assembly 8.

In yet another embodiment, a determination that the forceps, or any portion thereof, has reached the end of its useful life will permanently disable one or more components in the forceps. For example, a mechanical device may be configured to prevent further drive actuations or configured to prevent removal and/or replacement of a shaft and end effector 6. Device may also be permanently disabled by disabling an electrical component or electrically or mechanically severing an electrical connection, electrical trace or electrical wire thereby permanently disabling the capability of the forceps 10 to deliver electrosurgical energy. Mechanical indictor may be configured to disable the forceps by mechanically blocking the switch and preventing further activations of the switch 60.

In yet another embodiment of the present disclosure, an indicator system 200 as described herein is positioned in the shaft and end effector assembly 8 and operates independent of the housing assembly 6. A shaft and end effector assembly 8 with a use indicator system 200 would include an end effector activation indicator 230 with control circuitry 230a contained therein, the control circuitry 230a configured to disable the shaft and end effector assembly 8, or a suitable portion thereof, when the end-of-life has been reached. For example, the end-of-life may disable the flow of electrosurgical energy to the end effector assembly 100, short circuit the first and second jaw members 110, 120 thus generating a short circuit fault in the generator 500, open a connection to the first and second jaw members 110, 120 thus generating an open circuit fault in the generator 500, provide a signal to the housing assembly 6 or the generator 500 indicating a fault or provide an end-of-life signal to the housing assembly 6 or the generator 500. As such, the shaft and end effector assembly 8 including a use indicator system 200 may be used with a housing assembly 6 with or without a use indicator system 200.

In another embodiment, the indicator may indicate one or more end-of-life or near end-of-life conditions. For example, the indicator may initially provide a first color indicating the device is not in a near end-of-life condition (e.g., green indicator), a second color indicating the device is approaching a near end-of-life condition (e.g., yellow indicator), a third color indicating an end-of-life condition is eminent (e.g., flashing red) and a fourth color indicating the device has reached an end-of-life condition (e.g., solid red). One or more of the color-coded near end-of-life or end-of-life indicators may be used in conjunction with the numerical indicators discussed hereinabove. For example, the color of the numerical indicator or background color of the numerical indicator may indicate one or more end-of-life and/or near end-of-life conditions.

The present disclosure also relates to a method of performing an electrosurgical procedure including the steps of: providing an electrosurgical forceps 10 in an inoperable condition; making the forceps 10 operable by replacing a spent shaft and end effector assembly 8 (or a portion thereof, e.g., end effector assembly 100) with an unused shaft and end effector assembly 8 (or portion thereof); transferring information from the unused shaft and end effector assembly 8 (or portion thereof) to a control circuit 205 in the electrosurgical forceps 10; enabling the electrosurgical forceps 10 if the information from the unused shaft and end effector assembly 8 indicated the unused shaft and end effector assembly 8 was capable of performing electrosurgical procedures, and performing at least one electrosurgical procedure. The method may also include the step of displaying a value related to the information provided from the unused shaft and end effector assembly 8.

Another method of performing an electrosurgical procedure is illustrated in Figs. 4A-4C. The forceps 10, illustrate in Fig. 4A, is in an inoperable condition as indicated by the value of zero (0) displayed by the shaft and end effector activation indicator 230 on the shaft 12 of the shaft and end effector assembly 408a. The forceps 10 is disabled by the control circuit 205 of the use indicator system 200 (see Fig. 3) by interrupting the flow of electrosurgical energy from the generator 500. The interruption of electrosurgical energy may be accomplished by providing a fault signal to the generator 500, by disabling the switch 60, by interrupting the signal provided to the generator from the switch 60 and by interrupting the electrosurgical current path in the device or by any other suitable method of disabling an electrosurgical device.

As indicated by the handle activation indicator 210 and the shaft and end effector replacement indicator 220 the housing assembly 6 is capable of performing as many as 45 actuations (as displayed on the handle activation indicator 210) and capable of receiving up to three (3) additional unused shaft and end effector assembly 8 replacements (as displayed on the end effector replacement indicator 220).

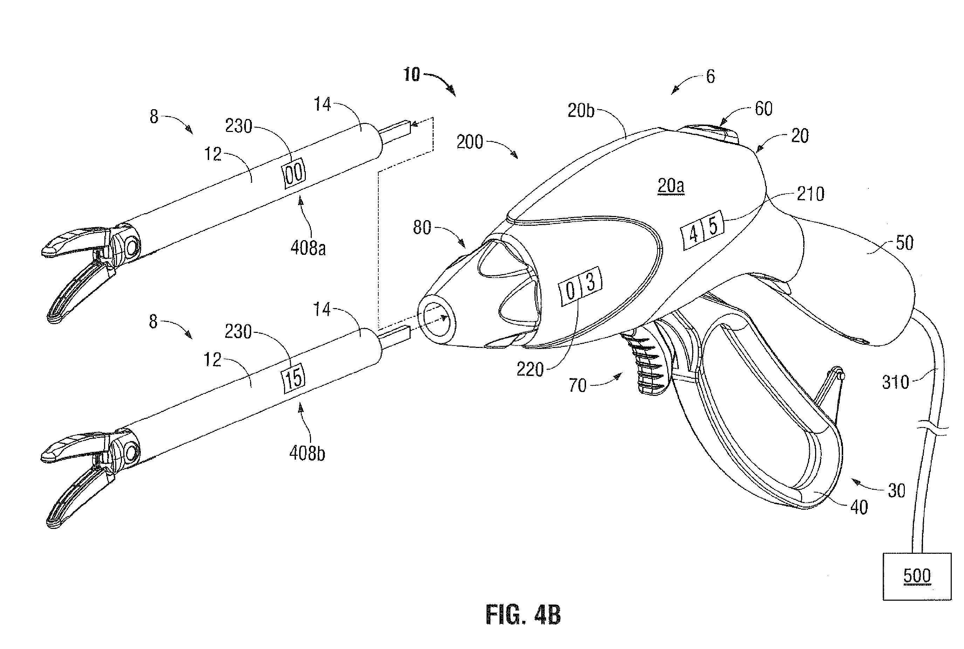

For example, in Fig. 4B the proximal end 14 of the spent shaft and end effector assembly 408a is removed from the rotating assembly 80. After removing the spend shaft and end effector assembly 408a, the proximal end 14 of an unused shaft and end effector assembly 408b is inserted into the rotating assembly 80. The shaft and end effector assembly sensor 340 (see Fig. 3) detects and reports the removal of the spent shaft and end effector assembly 408a and the subsequent insertion of an unused shaft and end effector assembly 408b to the control circuit 205 (see Fig. 3). The shaft and end effector replacement indicator 220 is adjusted by the control circuit 205 accordingly. As mentioned above, these adjustments may be implemented in strictly and electrical fashion, electro-mechanical fashion or a mechanical fashion.

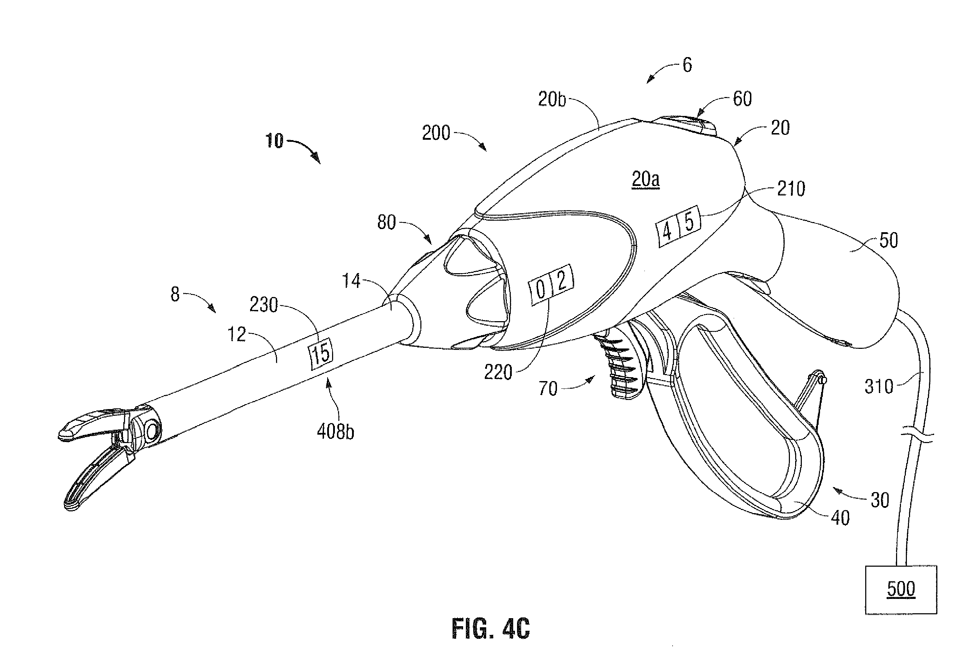

The forceps 10 illustrated in Fig. 4C is operational and the shaft and end effector activation indicator 230 indicates that as many as 15 activations may be performed with the unused shaft and end effector assembly 408b. The control circuit 205, having detected that an unused shaft and end effector assembly 408b was inserted into the housing assembly 6, decreases the shaft and end effector replacement indicator 220 from three (3) to two (2). As such, housing assembly 6 may received two additional shaft and end effector assemblies 408b before the housing assembly 6 is spent and is permanently disabled (due to the number of shaft and end effector assembly 408b replacements).

Yet another method of performing an electrosurgical procedure is illustrated in Figs. 5A-5C. The forceps 10, illustrated in Fig. 5A, is in an inoperable condition as indicated by the value of zero (0) displayed by the shaft and end effector activation indicator 230 on the shaft 12 of the shaft and end effector assembly 508. The forceps 10 is disabled by the control circuit 205 of the use indicator system 200 (see Figs. 1-3) by interrupting the flow of electrosurgical energy from the generator 500. Alternatively, the control circuit 205 may report the condition of the forceps 10 to the generator 500 thereby indirectly disabling the flow of electrosurgical energy.

As indicated by the handle activation indicator 210 and the shaft and end effector replacement indicator 220, the housing assembly 6 is capable of performing as many as fourteen (14) actuations (as displayed on the handle activation indicator 210) and capable of receiving up to three (3) additional unused end effector assemblies 100a (as displayed on the shaft and end effector replacement indicator 220).

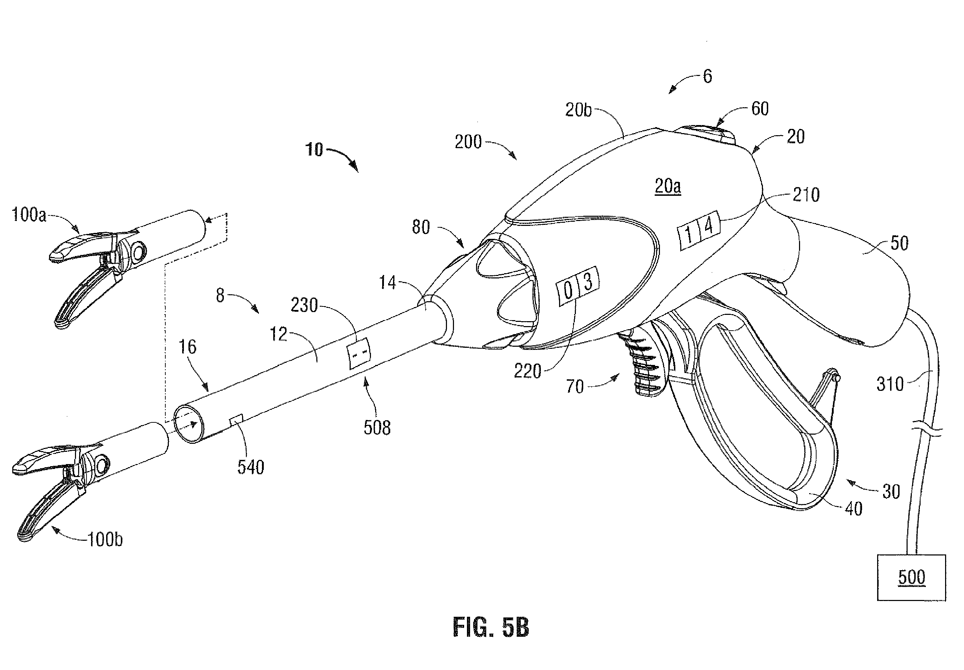

In Fig. 5B, the spent end effector assembly 100a is removed from the distal end 16 of the shaft 12. After removing the spent end effector assembly 100a, a used end effector assembly 100b is inserted into the distal end 16 of the shaft 12. The end effector assembly sensor 540 detects and reports the removal of the spent end effector assembly 100a and the subsequent insertion of the unused end effector assembly 100b to the control circuit 205 (see Fig. 3). The shaft and end effector replacement indicator 220 is adjusted by the control circuit 205 accordingly.

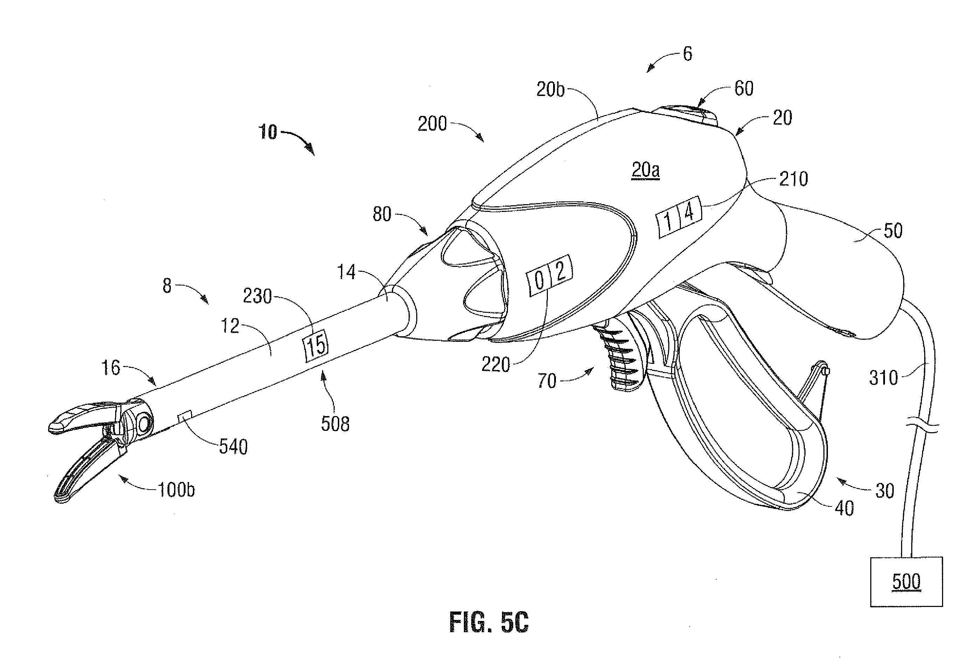

The forceps 10 illustrated in Fig. 5C is operational and the shaft and end effector activation indicator 230 indicates that as many as 15 activations may be performed with the unused end effector assembly 100b on the shaft and end effector assembly 508. Control circuit 205, having detected that an unused end effector assembly 100b was inserted into the housing assembly 6, decreased the end effector replacement indicator 220 from three (3) to two (2). As such, housing assembly 6 may receive two additional end effector assemblies 100b before the handle assembly is spent and permanently disabled (due to the replacement of the end effector assembly 100a).

The indicators on the forceps 10 illustrated in Fig. 5C indicate that the end effector assembly 100b is capable of 15 activations while the housing assembly 6 is capable of 14 activations. As such, if the conditions for "activation" are identical for the housing assembly 6 and the end effector assembly 100a, the handle activation indicator 210 will permanently disable the housing assembly 6 before the shaft and end effector activation indicator 230 reaches zero.

In an alternative method of performing a surgical procedure, the replacement of a spent shaft and end effector assembly 8 (or portion thereof) changes the number of activations provided in the handle activation indicator 210.

From the foregoing and with reference to the various figure drawings, those skilled in the art will appreciate that certain modifications can also be made to the present disclosure without departing from the scope of the same. While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

## Claims

1. An electrosurgical instrument, comprising:
a housing assembly having a first disposable treatment portion selectively attached thereto, the first disposable treatment portion adapted to connect to an electrosurgical generator that supplies energy thereto;
a control circuit disposed in electrical communication between the electrosurgical generator and the first disposable treatment portion, the control circuit being selectively actuatable to enable the flow of electrosurgical energy from the electrosurgical generator to the first disposable treatment portion; and
at least one indicator coupled to one of the housing assembly and the first disposable treatment portion, one of the at least one indicators configured to indicate a parameter related to the use of the electrosurgical instrument,
wherein the control circuit is configured to prevent activation of the electrosurgical instrument when the parameter of one of the at least one indicators indicates the useful life of the first disposable treatment portion has expired.

2. An electrosurgical instrument according to claim 1 wherein the first disposable treatment portion is an actuating jaw mechanism or an end effector assembly including an actuating jaw mechanism.

3. An electrosurgical instrument according to claim 1 or 2, wherein the parameter displayed by one of the at least one indicators is related to the actuations of the first disposable treatment portion or is related to the useful life of the housing assembly.

4. An electrosurgical instrument according to claim 1, 2 or 3, wherein the control circuit is configured to enable the electrosurgical instrument upon replacement of the first disposable treatment portion with an unused subsequent disposable treatment portion different than the first disposable treatment portion.

5. An electrosurgical instrument according to claim 1, 2, 3 or 4, wherein the at least one indicator includes:
a first indicator configured to indicate a first parameter related to the useful life of one of the first disposable treatment portion and one or more subsequent disposable treatment portions, and
a second indicator configured to indicate a second parameter related to the useful life of the housing assembly.

6. An electrosurgical instrument according to claim 5 wherein the second parameter is related to actuations of the electrosurgical instrument.

7. An electrosurgical instrument according to Claim 5 wherein the second parameter is related to replacements of the first disposable treatment portion and the subsequent disposable treatment portions, for example, the end-of-life of the housing assembly after a predetermined number of replacements exceeds a predetermined limit.

8. An electrosurgical instrument according to any one of the preceding claims wherein the control circuit includes at least one sensor configured to detect a mechanical actuation of one of a trigger assembly, a mechanical actuation of a jaw closure assembly and the removal of the first disposable treatment portion and the insertion of an unused subsequent disposable treatment portion different than the first disposable treatment portion.

9. An electrosurgical instrument according to any one of the preceding claims wherein the control circuit includes circuitry to disable the flow of electrosurgical energy, for example, to one of the first disposable treatment portion and the subsequent disposable treatment portion.

10. An electrosurgical instrument according to any one of the preceding claims wherein control circuit includes circuitry to disable the actuation signal provided to the electrosurgical generator.

11. An electrosurgical instrument according to any one of the preceding claims wherein the parameter is selected from a numerical indicator and a color indicator and/or is configured to indicate a near end-of-life condition, for example prior to indicating that the useful life of the first disposable treatment portion has expired.

12. An electrosurgical instrument according to claim 5, or an one of claims 6 to 11 as dependent on claim 5, wherein the electrosurgical instrument includes a third indicator, coupled to one of the housing assembly and the first disposable treatment portion, the third indicator configured to indicate a near end-of-life condition.

13. An electrosurgical instrument according to claim 12, wherein the third indicator indicates a near end-of-life condition prior to the first indicator or the second indicator indicating the expiration of useful life.

14. An electrosurgical instrument, comprising:
a housing assembly having a first disposable treatment portion selectively attached thereto, the first disposable treatment portion adapted to connect to an electrosurgical generator that supplies energy thereto;
a control circuit disposed in electrical communication between the electrosurgical generator and the disposable treatment portion, the control circuit being selectively actuatable to enable the flow of electrosurgical energy from the electrosurgical generator to the disposable treatment portion; and
at least one indicator coupled to the housing assembly, the indicator configured to indicate a parameter related to the use of the electrosurgical instrument,
wherein the control circuit is configured to prevent activation of the electrosurgical instrument when the parameter of one of the at least one indicators indicate the useful life of the housing assembly has expired.

15. An electrosurgical instrument, comprising:
a housing assembly adapted to connect to an electrosurgical generator that supplies electrosurgical energy thereto;
a first disposable treatment portion selectively attached to a distal end of the housing assembly and configured to receive electrosurgical energy therefrom, the first disposable treatment portion including:
an actuating jaw mechanism configured to delivery electrosurgical energy to tissue,
a control circuit disposed in the first disposable treatment portion and in electrical communication between the housing assembly and the actuating jaw mechanism, the control circuit being selectively actuatable to enable the flow of electrosurgical energy from the electrosurgical generator to the actuating jaw mechanism, and
at least one indicator coupled to the first disposable treatment portion and configured to indicate a parameter related to the use of the disposable treatment portion,
wherein the control circuit is configured to prevent activation of the electrosurgical instrument when the parameter of one of the at least one indicators indicate the useful life of the first disposable treatment portion has expired.
